(19) Europäisches Patentamt
European Patent Office
Office européen des brevets

(11) Veröffentlichungsnummer : **0 168 686**
**B1**

(12) # EUROPÄISCHE PATENTSCHRIFT

(45) Veröffentlichungstag der Patentschrift : **05.04.89**

(51) Int. Cl.⁴ : **C 07 D489/02**, C 07 D489/08// A61K31/485

(21) Anmeldenummer : **85107895.6**

(22) Anmeldetag : **26.06.85**

(54) **Verfahren zum N-demethylieren von Morphinan-alkaloiden.**

(30) Priorität : **17.07.84 HU 277484**

(43) Veröffentlichungstag der Anmeldung : **22.01.86 Patentblatt 86/04**

(45) Bekanntmachung des Hinweises auf die Patenterteilung : **05.04.89 Patentblatt 89/14**

(84) Benannte Vertragsstaaten : **AT BE CH DE FR GB IT LI NL SE**

(56) Entgegenhaltungen :
**DE-A- 2 254 298**
**DE-A- 2 727 805**
**GB-A- 1 166 798**

(73) Patentinhaber : **ALKALOIDA VEGYESZETI GYAR**
**Kabay János ut 27-29**
**H-4440 Tiszavasvári (HU)**

(72) Erfinder : **Hosztafi, Sándor, Dr.**
**Arpád ut 16**
**HU-4275 Monostorpályi (HU)**
Erfinder : **Timár, Tibor, Dr.**
**Kabay J. u. 17**
**HU-4440 Tiszavasvári (HU)**
Erfinder : **Nagy geb. Vajda, Julianna**
**Csokonai u. 16/a**
**HU-4440 Tiszavasvári (HU)**
Erfinder : **Fábián geb. Orbán, Ilona**
**Elmunkás ut 11**
**HU-4440 Tiszavasvári (HU)**

(74) Vertreter : **Lehn, Werner, Dipl.-Ing. et al**
**Hoffmann, Eitle & Partner Patentanwälte Arabellastrasse 4**
**D-8000 München 81 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 168 686 B1**

Jouve, 18, rue St-Denis, 75001 Paris, France

**Beschreibung**

Die Erfindung betrifft die Herstellung von Verbindungen der allgemeinen Formel (III)

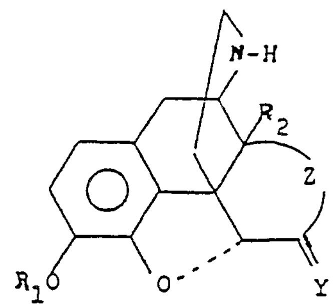

(III)

mit Morphinan-Struktur durch das N-Demethylieren von Morphinanalkaloiden der allgemeinen Formel (I).

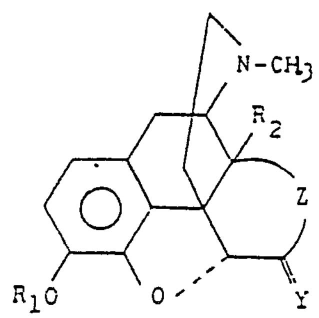

(I)

Der Abtausch der N-Methylgruppe der Morphinanalkaloide gegen eine andere Alkylgruppe führte von pharmakologischem und therapeutischem Gesichtspunkt zur Entdeckung bedeutender Morphinantagonisten sowie zum Trennen der agonistischen und antagonistischen Wirkung (Adv. in Biochem. Psychopharm. Vol. 8 : Narcotic Antagonists/Raven Press, New York 1974) ; E.F. Hahn : Drugs of the Future 9/6/, 443/1984/).

Da die Morphinanalkaloide (Morphin, Kodein, Tebain) aus natürlichen (pflanzlichen) Quellen zur Verfügung stehen, sind die meisten Verfahren bestrebt, sekundäre Amine aus den obigen N-Methyltertiär-aminen herzustellen. (Das sekundäre Amin kann im weiteren durch N-Alkylieren zur erwünschten Verbindung umgesetzt werden). Es ist zweckmäßig, die N-Methylgruppe im weiteren gegen eine leicht hydrolysierbare Funktionsgruppe auszutauschen.

Die Anforderungen an den N-demethylierenden Wirkstoff sind wie folgt : er soll billig, stabil, leicht herstellbar sein, über eine ausreichende Reaktivität verfügen, gute Ausbeuten sichern und nicht giftig sein. Das früher zum N-Demethylieren der Morphinanstruktur häufig eingesetzte Bromcyan (H.A. Hageman : Org. Reactions 7, 198/1953/) wurde in den letzten Jahren immer mehr von den Chlorameisensäureestern verdrängt. Der größte Nachteil von Bromcyan ist, daß es sehr giftig ist, wegen seiner Unstabilität zweckmäßig vor dem Verbrauch frisch hergestellt werden muß, andererseits erwähnen mehrere Autoren seine Explosivität.

Die Saure (oder alkalische) Hydrolyse der in der Reaktion nach von Braun entstehenden Cyanamide kann in bestimmten Fällen zum teilweisen Zerfall (oder Umsetzen) des Moleküls führen (A.C. Currie, G.T. New-bold, F.S. Spring : J. Chem. Soc. 4693/1961/) oder aber hält die Reaktion in der Harnstoffphase an (K.W. Bentley, D.G. Hardy : J. Am. Chem. Soc. 89, 3281/1967/). Im Gegensatz dazu können die Chlorameisensäureester leicht behandelt werden, sie sind weniger giftige Verbindungen, ihre Reinigung kann durch Destillieren gut gelöst werden. Die schon zu Beginn verwendeten Methyl-, Äthyl-, Phenyl-ester brachten beim N-Demethylieren ausgezeichnete Ausbeuten (85-95 %) (M.M. Abdel-Monem, P.S. Portoghese : J. Med. Chem. 15, 208/1972/ ; G.A. Brine, K.G. Boldt, C.K. Hart, F.I. Carroll : Organic Prep. Proced. Int. 8, 103/1976/ ; K.C. Rice, E.L. May : J. Heterocyclic Chem. 14, 665/1977/).

Es stellte sich heraus, daß das Isolieren der Intermediärurethan-Derivate nicht notwendig ist, sie können direkt durch eine Lauge oder Hydrazin weiterumgesetzt werden. Da die obigen Hydrolysen ziemlich langwierig sind (24-48 Stunden) und sich in dieser Zeit schon Zerfall und auch Nebenreaktionen abspielen können, wurden später für das N-Demethylieren Chlorameisensäure-2,2,2-trichlorethylester (I.J. Borowitz, V. Diakiw : J. Heterocyclic Chem. 12, 1123/1975/ ; J.I. De Graw, J.A. Lawson, J.L. Crase, H.L. Johnson, M. Ellis, E.T. Uyeno, G.H. Loew, D.S. Berkowitz : J. Med. Chem. 21, 415/1978/) und Chlorameisensäurevinylester vorgeschlagen.

Der Vorteil des Trichloräthylesters besteht darin daß er reaktiv ist und die entstehenden Urethane

durch aktiviertes Zinkpulver leicht gespalten werden können. Nachteilig ist jedoch, daß das Spalten der von den 14-Acyloxy-morphinanen erhaltenen Urethane durch Acylwandern O ⟶ N erfolgt (Ger. Offen. 2 727 805), was eine direkte Herstellung des N-Demethyl-Derivates nicht ermöglicht.

In der Fachliteratur gibt es auch ein Beispiel, wo der Zinkkomplex des N-Demethyl-Derivates entsteht (N.P. Peet : J. Pharm. Sci. 69, 1447/1980/). Nach den bisherigen Angaben aus der Fachliteratur ist der Chlorameisensäure-vinylester der idealste N-demethylierende Wirkstoff (U.S. Patentschriften Nr. 3 905 981, 4 141 897 und 4 161 597). Dieser Wirkstoff ist durch die elektronenanziehende Wirkung der Vinylgruppe sehr reaktiv, dadurch werden quantitative Reaktionen gesichert.

Wenn die Vinylurethane mit einer äquivalenten Säure (Salzsäure, Wasserstoffbromid) behandelt werden, spielt sich eine Addition ab, und mit Methanol gesiedet entsteht das Salz des sekundären Amins. Das Spalten des Urethans erfolgt also unter sehr milden Reaktionsbedingungen, und so kann man das sekundäure Amin mit guter Ausbeute und großer Reinheit erhalten.

Die aus 14-O-Acyl-morphinanen entstehenden Urethane werden mit Säure behandelt, und das Salz (salzsäures, wasserstoffbromidhaltiges) der Verbindung 14-O-Acyl-N-demethyl entsteht, in diesem Fall gibt es also kein O ⟶ N Acylwandern. Neben den erwähnten Vorteilen muß jedoch erwähnt werden, daß die Herstellung von Chlorameisensäurevinylester schwer zu lösen ist (wegen der Pyrolyse), was das Studieren von weiteren N-Demethylierungsmethoden erforderlich macht. Unsere Wahl fiel auf Diphosgen, da es für das N-Demethylieren von Tropanalkaloiden erfolgreich eingesetzt wurde. Das Diphosgen oder der Chlorameisensäure-tri-chlormethylester kann durch das Chlorieren von Chlorameisensäuremethylester leicht hergestellt werden (A. Efraty, I. Feinstein, L. Wackerle, A. Goldman : J. Org. Chem. 45, 4059 /1980/ ; K. Kurita, Y. Iwakura : Org. Synth. 59, 195/1980/).

Da es sich in den meisten Reaktionen ähnlich wie Phosgen verhält (ein Mol Diphosgen liefert 2 Mol Phosgen), sein Siedepunkt 128 °C beträgt, seine Tension 0,0133 bar (10 mmHg) bei Raumtemperatur niedrig ist, ist man neuerdings bestrebt, das sehr giftige Phosgen durch Diphosgen zu ersetzen. Das Diphosgen und das Phosgen reagieren mit tertiären Aminen ähnlich wie Chlorameisensäureester, die Methylgruppe wird in Form von Methylchlorid abgespalten und Carbamoylchlorid entsteht, bei Diphosgen entsteht auch Phosgen (H. Babad, A.D. Zeiler : Chem. Rev. 73, 75 /1973/). Das Phosgen wurde zum N-Demethylieren von tertiären Aminen als Analog der von Braun-Reaktion schon im 1947 vorgeschlagen (V.A. Rudenko, A.Y. Jakubovich, T.Y. Nikiforova : J. Gen. Chem. (USSR) 17, 2256/1947/ ; C.A. 42, 4918e).

Da das Phosgen mit tertiären Aminen Amin-Phosgen-Komplexe im Verhältnis 1 : 1 und 1 : 2 bilden kann und beim Zerfall der letzteren ein tertiäres Amin entsteht (H. Bahad, A.D. Zeiler : Chem. Rev. 73, 75 /1973/), ist die Anwendung von Phosgenüberschuß zweckmäßig, da sich auch tetrasubstituierter Harnstoff bildet, wenn sich das Amin im Überschuß befindet.

Banholzer et al. (in den britischen Patentschriften Nr. 1 166 798 und 1 167 688 und R. Banholzer, A. Heusner, W. Schulz : Liebigs Ann. Chem. 2227/1975/) erwähnten als erste das N-Demethylieren von Alkaloiden mit Tropanstruktur mit Phosgen und Diphosgen. Das erhaltene Carbamoylchlorid (es muß nicht isoliert werden) hydrolysiert mit Wasser erwärmt zu sekundärem Aminchlorhydrat und Kohlendioxyd.

Dementsprechend wurde zweckmäßig das N-Demethylieren von Verbindungen mit Morphinanstruktur mit Diphosgen untersucht, da voraussichtlich das Hydrolysieren der Carbamoylchloride unter viel milderen Bedingungen als das der Urethane oder Cyanamide erfolgt.

Als Modellverbindung wurde Dihydrokodeinon gewählt und festgestellt, daß es mit Diphosgen in einer 1,2-Dichloräthan (oder Dichlormethan, Chloroform, Tetrachlorkohlenstoff, Benzol, Toluol) enthaltenden Lösung das erwartete N-Chlorcarbonyl-N-demethyl-dihydrokodeinon liefert.

Die Struktur des Letzteren wurde auch dadurch bestätigt, daß das Dihydrokodeinon mit Phosgen eine ähnliche Reaktion aufwies, andererseits, daß sich, wenn man die Chlorcarbonyl-Verbindung mit Äthanol siedet, das in der Fachliteratur beschriebene Urethan, das N-Äthoxy-carbonyl-N-demethyl-dihydrokodeinon bildete (G. Horváth, P. Kerekes, Gy. Gaál, R. Bognár : Acta Chim. Acad. Sci. Hung. 82, 217/1974/). Die Bildung des N-Chlorcarbonyl-Derivates wird durch wasserfreies Natriumcarbonat katalysiert sowie ist es wegen der Bildung von eventuellen Säurespuren ebenfalls zweckmäßig, wasserfreies Natriumcarbonat anzuwenden.

Im weiteren kann das N-Chlorcarbonyl-Derivat mit Wasser erwärmt zu N-Demethyl-dihydrokodeinonchlorhydrat hydrolysiert werden.

Das Diphosgen und Phosgen acylieren die alkohol- und phenolhaltigen Hydroxygruppen während Bildung von Salzsäure, und die Salzsäure bindet das tertiäre Amin, deshalb ist es zweckmäßig, die die Hydroxylgruppe enthaltenden Morphin-Derivate durch Acylieren zu schützen.

In der DE-A-2 254 298 wird die Herstellung von Heteroaryl-normorphine-Verbindungen beschrieben. Es wird dort dargelegt, daß man für die Herstellung des als Ausgangsverbindungen dienenden Normorphonins Heroin mit Phosgen umsetzen kann und das gebildete N-Chlorcarbonylnorheroin dann mit Wasser verkocht.

Das vorliegende Verfahren ist zum N-Demethylieren von Dihydrokodeinon, Dihydromorphinon, Kodein, Dihydrokodein, Morphin, Dihydromorphin, Äthylmorphin, Dihydroäthylmorphin, Desoxykodein-E, Dihydrodesoxykodein-E, 14-Hydroxy-dihydrokodeinon, 14-Hydroxykodeinon, 14-Hydroxy-dihydromorphinon, 3-O-Benzylmorphin, Azidomorphin geeignet.

Die Erfindung betrifft ein Verfahren zur Herstellung von Verbindungen mit N-Desmethyl-morphinan-

Struktur der allgemeinen Formel (III),

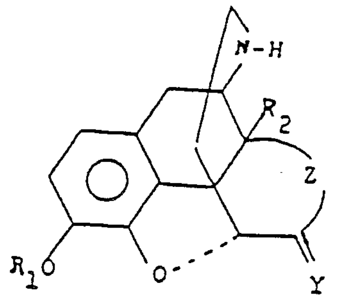

(III)

worin

Z $CH_2$—$CH_2$ oder CH=CH ist,

Y für ein Sauerstoffatom oder die Gruppe $R_3$ und $R_4$ steht, worin

$R_3$ ein Wasserstoffatom, eine Hydroxylgruppe, eine Acyloxy-Gruppe mit 1-3 Kohlenstoffatomen oder eine Benzoyloxy-Gruppe bedeutet, seine Raumstellung $\alpha$ ist,

$R_4$ ein Wasserstoffatom, Halogenatom, eine Hydroxylgruppe, eine Alkylgruppe mit 1-3 Kohlenstoffatomen, eine Acyloxy-Gruppe mit 1-3 Kohlenstoffatomen, eine Benzoyloxy- oder Azidogruppe ist, seine Raumstellung $\beta$ ist,

$R_2$ für ein Wasserstoffatom, eine Acyloxy-Gruppe mit 1-3 Kohlenstoffatomen oder eine Benzoyloxy-Gruppe steht,

$R_1$ ein Wasserstoffatom, eine Alkylgruppe mit 1-5 Kohlenstoffatomen, eine Aryl- oder Aralkylgruppe, eine Acylgruppe mit 1-3 Kohlenstoffatomen oder eine Benzoylgruppe bedeutet,

unter der Voraussetzung, daß

wenn

$R_3$ für ein Wasserstoffatom, eine Hydroxylgruppe, Acyloxy- oder Benzoyloxy-Gruppe steht,

$R_4$ ein Wasserstoffatom oder eine Alkylgruppe ist und

wenn

$R_4$ ein Wasserstoffatom, Halogenatom, eine Hydroxylgruppe, Acyloxy-, Benzoyloxy- oder Azidogruppe bedeutet,

$R_3$ ein Wasserstoffatom ist

durch Umsetzen von Morphinan-alkaloiden der allgemeinen Formel (I)

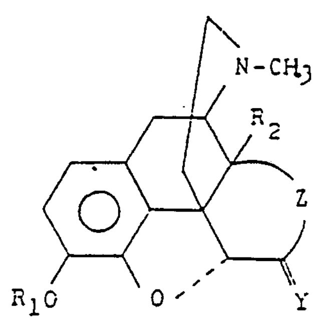

(I)

worin Z, Y, $R_1$ und $R_2$ wie oben definiert sind — mit Phosgen oder Diphosgen bei einer Temperatur von 0-120 °C in einem Lösungsmittel und anschließender Hydrolyse des entstehenden N-Chlor-carbonyl-N-desmethyl-Derivats der allgemeinen Formel (II)

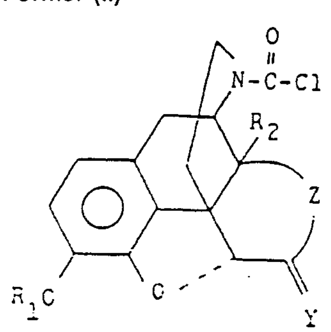

(II)

worin Z, Y, $R_1$ und $R_2$ die vorher angegebene Bedeutung haben mit einer Mineralsäure, daß dadurch

gekennzeichnet ist, daß man die Umsetzung mit Phosgen oder Diphosgen in 1,2-Dichlorethan in Gegenwart von Alkalicarbonaten oder Alkalihydrogencarbonaten durchführt und dann, ohne Isolierung, das entstandene N-Chlor-carbonyl-N-desmethyl-Derivat der allgemeinen Formel (II) mit 5 % Salzsäure erwärmt.

Die Raumstellung der Gruppen $R_3$ und $R_4$ ist in Abhängigkeit der Bedeutung der Gruppe Z wie folgt : wenn Z für $CH_2—CH_2$ steht, dann ist die Raumstellung der Gruppe $R_3$ axial und die Raumstellung der Gruppe $R_4$ äquatorial ; steht Z für CH=CH, dann ist die Raumstellung der Gruppe $R_3$ quasi-äquatorial und die der Gruppe $R_4$ quasi-axial.

Der Erfindung liegt die eine wichtige Erkenntnis zugrunde, daß die entsprechenden N-Chlorcarbonyl-N-demethyl-Derivate unter den bisher bekannten mildesten und einfachsten Reaktionsbedingungen abhydrolysiert werden können, das heißt Zerfall und schädliche Nebenreaktionen spielen sich nicht ab, beim Verarbeiten entstehen keine giftigen Mutterlaugen und Abwässer.

Die Anwendung von Phosgen und Diphosgen ist im Vergleich zu Bromcyan und Chlorameisensäureestern sowohl hinsichtlich der Gefährlichkeit der Reagenzien, der Schwierigkeit ihrer Herstellung, ihres Preises und ihrer Beschaffbarkeit für Verfahren in Industriemaßstäben am vorteilhaftesten. Beim erfindungsgemäßen Verfahren wird die Anwendung von Diphosgen im Vergleich zum giftigen Phosgen als vorteilhafter betrachtet, wobei berücksichtigt wird, daß das bei der Reaktion von Diphosgen entstehende Phosgen aus dem System durch Spülen mit Stickstoff durch einen geeigneten Wäscher sicher entfernt werden kann.

Beim erfindungsgemäßen Verfahren ist das Molverhältnis der Morphinan-alkaloide der allgemeinen Formel (I) und von Phosgen/Diphosgen vorteilhaft 1 : 3 bis 1 : 20, insbesondere 1 : 10 und das der Morphinan-alkaloide der allgemeinen Formel (I) und der Alkali-carbonate/Alkalihydrocarbonate 1 : 0,5 bis 1 : 2 und vorzugsweise 1 : 1.

Die Umsetzung mit Diphosgen wird vorzugsweise bei einer Temperatur von 20 bis 120 °C, insbesondere 80 °C durchgeführt.

Geeignete Alkalicarbonate sind Natriumcarbonat oder Kaliumcarbonat und geeignete Alkalihydrogencarbonate sind Natriumhydrogencarbonat oder Kaliumhydrogencarbonat.

Das erfindungsgemäße Verfahren ist zum wirtschaftlichen Herstellen der von pharmakologischem und therapeutischem Gesichtspunkt wichtigen N-Demethylmorphinan-Derivate in Industriemaßstäben geeignet.

Die vorliegende Erfindung wird im weiteren durch Beispiele ausführlicher veranschaulicht, ohne sie auf die Beispiele einzuschränken.

Die Schmelzpunkte wurden in einem Koffler-Apparat bestimmt, die Angaben sind nicht korrigiert. Für die Dünnschicht-chromatographie-Untersuchungen wurden MERCK 5 554 Silicagel $60F_{254}$ Folien verwendet. Eluenten : Benzol : Methanol = 8 : 2 ; Chloroform : Methanol = 9 : 1 beziehungsweise Chloroform : Aceton : Äthylamin = 5 : 4 : 1/V/V/. Die Flecken wurden mit UV-Licht und einem Dragendorff-Reagenten detektiert. NMR-Aufnahmen wurden in einem Apparat von Typ Bruker W200SY bei 200 MHz hergestellt. Die chemischen Verschiebungen wurden in δ (ppm) angegeben. Die Massenspektren wurden im Apparat VG-7035/GC-MS-DS/ durch Elektronenstoßionisation hergestellt.

## Beispiele

### 1. N-Demethyl-dihydrokodeinon

3 g (10 mM) Dihydrokodeinon werden in 60 ml 1,2-Dichloräthan gelöst, 1 g (10 mM) Natriumcarbonat wird zugesetzt und unter Rühren bei 0 °C werden 19,8 g (12 ml, 100 mM) Diphosgen dazugetropft. Das Gemisch wird 30 Minuten bei 0 °C gerührt, dann läßt man es auf Raumtemperatur erwärmen und siedet es 10 Stunden lang unter Rühren. Es wird mit Stickstoff durchgespült, das anorganische Salz wird abgefiltert.

Mit 3 × 20 ml 5 %iger Salzsäure (eiskalt) wird die 1,2-Dichloräthan enthaltende Lösung gewaschen, dann die organische Phase in Vakuum eingedampft. Dem Rückstand werden 100 ml Wasser zugesetzt und auf einem Wasserbad 5 Stunden lang erwärmt. Er wird abgekühlt und unter 10 °C mit konzentriertem Ammoniumhydroxid alkalisiert (pH = 9-10). Nach Extraktion mit Chloroform (3 × 50 ml) wird die organische Phase mit Salzwasser gewaschen und über Magnesiumsulfat getrocknet.

Ausbeute : 2,33 g (82 %) Schm. p. : 150-151 °C (Äthylacetat)

PMR ($CDCl_3$) : 6,66 dd (H-1,2 ; 2H) ; 4,7 s (H-5β ; 1H) ; 3,9 s ($OCH_3$ ; 3H).

MS : m/e 285 ($M^-$ ; 23 %)

Aus der 5 %igen salzsäuren Lösung kann man durch Alkalisieren (konzentriertes Ammoniumhydroxid) und Extraktion (Chloroform) 0,4 g (13 %) Dihydrokodeinon rückgewinnen.

### 2. N-Demethyl-dihydro-morphinon

3,3 g (10 mM) 3-O-Acetyl-dihydromorphinon werden in 60 ml 1,2-Dichloräthan gelöst, 1 g (10 mM) Natriumcarbonat wird zugesetzt und unter Rühren werden 19,8 g (12 ml, 100 mM) Diphosgen bei 0 °C dazugetropft. Das Gemisch wird 30 Minuten bei 0 °C gerührt, dann läßt man es auf Raumtemperatur

erwärmen und siedet es 15 Stunden lang unter Rühren. Es wird mit Stickstoff gespült, das anorganische Salz wird abgefiltert. Es wird mit 3 × 20 ml 5 %iger Salzsäure gewaschen, dann wird die organische Phase in Vakuum eingedampft. Dem Rückstand werden 100 ml Wasser zugesetzt, und er wird 8 Stunden gesiedet. Er wird unter 10 °C gekühlt, das Chlorhydrat des Produktes wird kristallin abgeschieden. Es wird gefiltert und mit kaltem Wasser gewaschen.

Ausbeute : 2,15 g (70 %), Chlorhydrat

Schmelzpunkt der daraus rückgewonnenen Base : 300-303 °C.

Der Ausgangsstoff wird wie in Beispiel 1 aus der 5 %igen salzsäuren Lösung rückgewonnen.

3. N-Demethyl-kodein

3,41 g (10 mM) 6-O-Acetyl-kodein werden in 80 ml 1,2-Dichloräthan gelöst, 1 g (10 mM) Natriumcarbonat wird zugesetzt und unter Rühren werden 19,8 g (12 ml, 100 mM) Diphosgen bei 0 °C dazugetropft. Das Gemisch wird 30 Minuten lang bei 0 °C gerührt, dann läßt man es auf Raumtemperatur erwärmen und rührt und siedet es 20 Stunden lang. Es wird mit Stickstoff gespült, das anorganische Salz wird abgefiltert. Die organische Phase wird mit 3 × 20 ml 5 %iger Salzsäure gewaschen, dann eingedampft. Der Rückstand wird mit 60 ml 5 %iger Salzsäure 5 Stunden lang gesiedet. Es wird unter 10 °C gekühlt, das Produkt kristallin abgeschieden, abgefiltert, mit Eiswasser gewaschen. Das kristalline Chlorhydrat wird im Wasser gelöst, mit konzentriertem Ammoniumhydroxid alkalisiert, mit Chloroform extrahiert, die chloroformhaltige Phase wird getrocknet und eingedampft. Die so gewonnene Base wird aus Aceton oder Äthylacetat kristallisiert.

Ausbeute : 2,3 g (80 %) Schm. p. : 185 °C

PMR ($CDCl_3$) : 6,65 dd (H-1,2 ; 2H) ; 5,7 m (H-8 ; 1H) ; 5,3 m (H-7 ; 1H) ; 4,9 d (H-5β ; 1H) ; 4,2 m (H-6β ; 1H) ; 3,8 s (OMe ; 3H).

MS : m/e 285 ($M^+$ ; 100 %), 215 (50 %)

4. N-Demethyl-dihydrokodein

3,34 g (10 mM) 6-O-Acetyl-dihydrokodein werden wie in Beispiel 3 beschrieben N-demethyliert. Nach der sauren Hydrolyse scheidet sich durch Kühlen das Salzsäure-Salz des Produktes nicht aus der Lösung ab, deshalb wird es — direkt mit konzentriertem Ammoniumhydroxid alkalisiert — durch Extraktion mit Chloroform gewonnen und aus Äthanol kristallisiert.

Ausbeute : 1,9 g (66 %) Schm. p. : 194 °C

PMR ($CDCl_3$) : 6,7 dd (H-1,2 ; 2H) ; 4,6 d (H-5β ; 1H) ; 4,1 m (H-6β ; 1H) ; 3,8 s (OMe ; 3H)

MS : m/e 287 ($M^+$ ; 100 %)

5. N-Demethyl-morphin

3,7 g (10 mM) 3,6-Di-O-Acetyl-morphin werden in 100 ml 1,2-Dichloräthan gelöst und wie in Beispiel 3 beschrieben N-demethyliert. Nach dem Abkühlen der salzsäuren Lösung wird der pH-Wert mit konzentriertem Ammoniumhydroxid auf 9, eingestellt. Das Produkt scheidet sich kristallin ab, es wird abgefiltert und mit kaltem Wasser gewaschen.

Ausbeute : 2,1 g (77 %) ; erste Generation 1,8 g ; aus der alkalischen Lösung Chloroform-isopropanol (3 : 1, v/v) anwendent durch Extraktion 0,3 g zweite Generation.

Schm. p. : 274-7 °C

PMR (DMSO-$d_6$) : 6,4 dd (H-1,2 ; 2H) ; 5,6 m (H-8 ; 1H) ; 5,2 m (H-7 ; 1H) ; 4,7 dd (H-5β ; 1H) ; 4,1 m (H-6β ; 1H)

MS : m/e 271 ($M^+$ ; 100 %) ; 201 (51 %)

6. N-Demethyl-dihydromorphin

3,71 g (10 mM) 3,6-Di-O-acetyl-dihydromorphin werden in 100 ml 1,2-Dichloräthan gelöst und wie in Beispiel 5 beschrieben demethyliert.

Ausbeute : 1,65 g (60 %) Schm. p. : 262-6 °C

PMR (DMSO-$d_6$) : 6,5 dd (H-1,2 ; 2H) ; 4,2 d (H-5β ; 1H) ; 3,8 m (H-6β ; 1H)

MS : m/e 273 ($M^+$ ; 100 %) ; 228 (22 %) ; 150 (45 %).

7. N-Demethyl-morphin-3-äthyläther

3,55 g (10 mM) 6-O-Acetyl-äthylmorphin werden N-demethyliert wie in Beispiel 3 beschrieben. Das Chlorhydrat des Produktes scheidet sich durch Kühlen ab, wird gefiltert und mit kaltem Wasser gewaschen. Daraus wird die Base rückgewonnen, aus Äthanol kristallisiert.

Ausbeute : 2,5 g (84 %) Schm. p. : 156-7 °C.

8. N-Demethyl-dihydromorphin-3-äthyläther

3,57 g (10 mM) 6-O-Acetyl-dihydroäthylmorphin wird wie in Beispiel 4 beschrieben N-demethyliert. Aus der salzsäuren Lösung wird das Produkt durch Alkalisieren und Extraktion gewonnen und aus Äthylacetat kristallisiert.

Ausbeute : 2,32 g (77 %) Schm. p. : 128-131 °C

PMR ($CDCl_3$) : 6,6 dd (H-1,2 ; 2H) ; 4,5 d (H-5β ; 1H) ; 4,1 m (O—<u>CH₂</u>—CH₃ ; 2H) ; 3,95 m (H-6β) ; 1H) ; 1,4 t (O—CH₂—<u>CH₃</u> ; 3H)

MS : m/e 301 ($M^+$ ; 100 %).

9. N-Demethyl-desoxykodein-E

1,42 g (5 mM) Desoxykodein-E werden in 50 ml 1,2-Dichloräthan gelöst. Man setzt 0,5 g (5 mM) Natriumcarbonat zu und zu der erhaltenen Suspension werden unter Rühren und Kühlen 6,0 ml (50 mM) Diphosgen getropft. Nach 30 Minuten Rühren wird das Kühlen eingestellt, dann wird das Gemisch 15 Stunden lang unter Rühren gesiedet. Nach dem Abkühlen wird mit Stickstoff gespült, das anorganische Salz abgefiltert und die organische Phase mit 2 × 20 ml kalter 5 %iger Salzsäure und salzigem Wasser gewaschen. Das Dichloräthan wird im Vakuum eingedampft, und dem Rückstand werden 60 ml Wasser zugesetzt und auf einem Wasserbad 8 Stunden lang erwärmt. Nach Abgekühlen wird es alkalisiert (pH = 10 ; konzentriertes $NH_4OH$) und mit 3 × 30 ml Chloroform extrahiert. Nach dem Trocknen des Chloroform-Phase (Magnesiumsulfat) erhält man durch Vakuumeindampfen das N-Demethyl-desoxykodein-E (0,6 g, 60 %), das dünnschichtchromatographisch homogen ist. Die Base wird nicht kristallin, aber wenn ihre alkoholische Lösung mit 48 %igem Wasserstoffbromid angesäuert wird, erhält man kristallines Hydrobromid.

Schmelzpunkt : 307-310 °C. (Schmalzpunkt nach der Fachliteratur : 311-312 °C, R.L. Clark et al. : J. Am. Chem. Soc. 75, 4963/1953/).

10. N-Demethyl-dihydrodesoxykodein-E

Man geht von 1,4 g (5 mM) Dihydrodesoxykodein-E wie in Beispiel 9 aus und erhält nach der Extraktion mit Chloroform 0,45 g (33 %) kristallines N-Demethyl-Dihydrodesoxykodein-E. Kristallisiert aus Äthylacetat beträgt der Schmelzpunkt 91-92 °C (Schmelzpunkt in der Fachliteratur : 92-94 °C ; R.L. Clark et al. : J. Am. Chem. Soc. 75, 4963/1953/).

**Patentansprüche**

1. Verfahren zur Herstellung von Verbindungen mit N-Desmethyl-morphinan-Struktur der allgemeinen Formel (III),

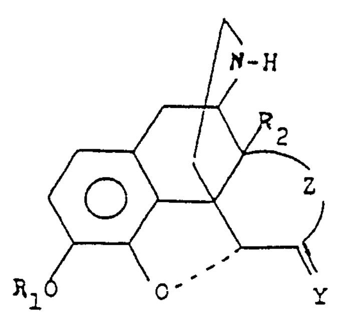

(III)

worin

Z $CH_2$—$CH_2$ oder CH=CH ist,

Y für ein Sauerstoffatom oder die Gruppe $R_3$ und $R_4$ steht, worin

$R_3$ ein Wasserstoffatom, eine Hydroxylgruppe, eine Acyloxy-Gruppe mit 1-3 Kohlenstoffatomen oder eine Benzoyloxy-Gruppe bedeutet, seine Raumstellung α ist,

$R_4$ ein Wasserstoffatom, Halogenatom, eine Hydroxylgruppe, eine Alkylgruppe mit 1-3 Kohlenstoffatomen, eine Acyloxy-Gruppe mit 1-3 Kohlenstoffatomen, eine Benzoyloxy- oder Azidogruppe ist, seine Raumstellung β ist,

$R_2$ für ein Wasserstoffatom, eine Acyloxy-Gruppe mit 1-3 Kohlenstoffatomen oder eine Benzoyloxy-Gruppe steht,

$R_1$ ein Wasserstoffatom, eine Alkylgruppe mit 1-5 Kohlenstoffatomen, eine Aryl- oder Aralkylgruppe, eine Acylgruppe mit 1-3 Kohlenstoffatomen oder eine Benzoylgruppe bedeutet,

unter der Voraussetzung, daß

wenn

$R_3$ für ein Wasserstoffatom, eine Hydroxylgruppe, Acyloxy- oder Benzoyloxy-Gruppe steht,

$R_4$ ein Wasserstoffatom oder eine Alkylgruppe ist und
wenn
$R_4$ ein Wasserstoffatom, Halogenatom, eine Hydroxylgruppe, Acyloxy-, Benzoyloxy- oder Azidogruppe bedeutet,
$R_3$ ein Wasserstoffatom ist
durch Umsetzen von Morphinan-alkaloiden der allgemeinen Formel (I)

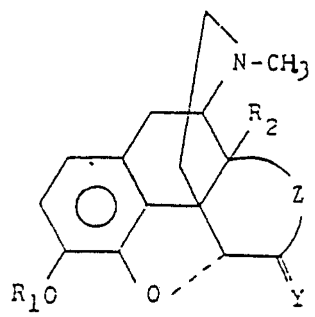
(I)

worin Z, Y, $R_1$ und $R_2$ wie oben definiert sind — mit Phosgen oder Diphosgen bei einer Temperatur von 0-120 °C in einem Lösungsmittel und anschließender Hydrolyse des entstehenden N-Chlor-carbonyl-N-desmethyl-Derivats der allgemeinen Formel (II)

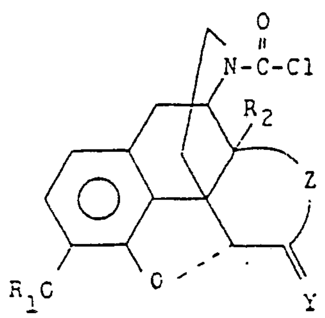
(II)

worin Z, Y, $R_1$ und $R_2$ die vorher angegebene Bedeutung haben mit einer Mineralsäure, dadurch gekennzeichnet, daß man die Umsetzung mit Phosgen oder Diphosgen in 1,2-Dichlorethan in Gegenwart von Alkalicarbonaten oder Alkalihydrogencarbonaten durchführt und dann, ohne Isolierung, das entstandene N-Chlor-carbonyl-N-desmethyl-Derivat der allgemeinen Formel (II) mit 5 % Salzsäure erwärmt.

2. Verfahren nach Anspruch 1, dadurch gekennzeichnet, daß das Molverhältnis der Morphinan-alkaloide der allgemeinen Formel (I) und von Phosgen/Diphosgen 1 : 3-1 : 20, zweckmäßig 1 : 10 und das der Morphinan-alkaloide der allgemeinen Formel (I) und der Alkali-carbonate/Alkali-hydrocarbonate 1 : 0,5-1 : 2, zweckmäßig 1 : 1 beträgt.

3. Verfahren nach Anspruch 1, dadurch gekennzeichnet, daß die Reaktion mit Diphosgen bei einer Temperatur von 20-120 °C, vorzugsweise 80 °C erfolgt.

4. Verfahren nach Anspruch 1, dadurch gekennzeichnet, daß man als Alkalicarbonat, Natriumcarbonat oder Kaliumcarbonat und als Alkalihydrogencarbonat, Natriumhydrogencarbonat oder Kaliumhydrogencarbonat verwendet.

**Claims**

1. Process for the preparation of compounds having the N-demethylmorphinane structure of the general formula (III)

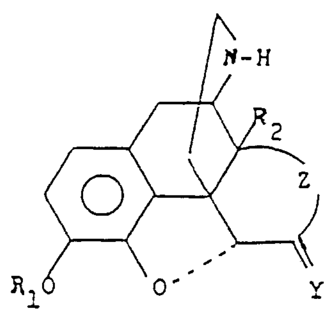
(III)

wherein

Z is $CH_2—CH_2$ or CH=CH,

Y is an oxygen atom or a group $R_3$ and $R_4$ wherein

$R_3$ is a hydrogen atom, a hydroxy group, an acyloxy group having from 1 to 3 carbon atoms or a benzoyloxy group, its spatial configuration being α,

$R_4$ is a hydrogen atom, a halogen atom, a hydroxy group, an alkyl group having from 1 to 3 carbon atoms, an acyloxy group having from 1 to 3 carbon atoms, a benzoyloxy or azido group, its spatial configuration being β,

$R_2$ is a hydrogen atom, an acyloxy group having from 1 to 3 carbon atoms or a benzoyloxy group,

$R_1$ is a hydrogen atom, an alkyl group having from 1 to 5 carbon atoms, an aryl or aralkyl group, an acyl group having from 1 to 3 carbon atoms or a benzoyl group,

with the proviso that

when

$R_3$ is a hydrogen atom, a hydroxy group, an acyloxy or benzoyloxy group,

$R_4$ is a hydrogen atom or an alkyl group, and

when

$R_4$ is a hydrogen atom, a halogen atom, a hydroxy group, an acyloxy, benzoyloxy or azido group,

$R_3$ is a hydrogen atom,

by reaction of morphinane alkaloids of the general formula (I)

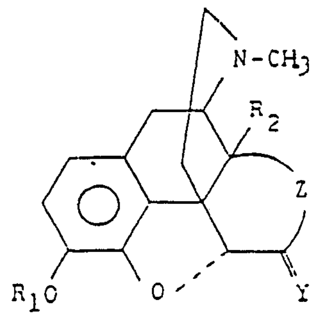

(I)

in which Z, Y, $R_1$ and $R_2$ have the definitions given above, with phosgene or diphosgene at a temperature of from 0 to 120 °C in a solvent, and subsequent hydrolysis of the resulting N-chlorocarbonyl-N-demethyl derivative of the general formula (II)

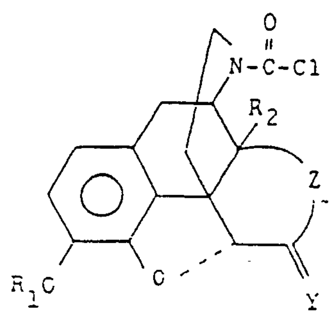

(II)

in which Z, Y, $R_1$ and $R_2$ have the meanings given above, with a mineral acid, which process is characterised in that the reaction with phosgene or diphosgene is carried out in 1,2-dichloroethane in the presence of alkali metal carbonates or alkali metal hydrogen carbonates, and then, without isolation, the resulting N-chlorocarbonyl-N-demethyl derivative of the general formula (II) is heated with 5 % hydrochloric acid.

2. Process according to claim 1, characterised in that the molar ratio of the morphinane alkaloids of the general formula (I) to phosgene/diphosgene is from 1 : 3 to 1 : 20, advantageously 1 : 10, and that of the morphinane alkaloids of the general formula (I) to the alkali metal carbonates/alkali metal hydrogen carbonates is from 1 : 0.5 to 1 : 2, advantageously 1 : 1.

3. Process according to claim 1, characterised in that the reaction with diphosgene is carried out at a temperature of from 20 to 120 °C, preferably 80 °C.

4. Process according to claim 1, characterised in that sodium carbonate or potassium carbonate is used as the alkali metal carbonate, and sodium hydrogen carbonate or potassium hydrogen carbonate is used as the alkali metal hydrogen carbonate.

## Revendications

1. Procédé de préparation de composés à structure de N-desméthyl-morphinane de formule générale (III),

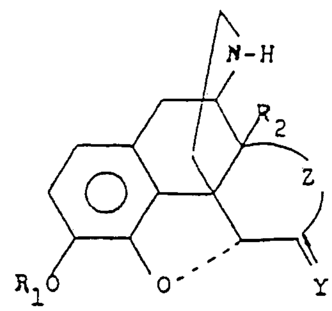

(III)

où

Z représente $CH_2—CH_2$ ou CH=CH,

Y représente un atome d'oxygène ou le groupe $R_3$ et $R_4$, où

$R_3$ représente un atome d'hydrogène, un groupe hydroxyle, un groupe acyloxy en $C_1$ à $C_3$ ou un groupe benzoyloxy, sa position dans l'espace est α,

$R_4$ représente un atome d'hydrogène, un atome d'halogène, un groupe hydroxyle, un groupe alcoyle en $C_1$ à $C_3$, un groupe acyloxy en $C_1$ à $C_3$, un groupe benzoyloxy ou azido, sa position dans l'espace est β,

$R_2$ représente un atome d'hydrogène, un groupe acyloxy en $C_1$ à $C_3$ ou un groupe benzoyloxy,

$R_1$ représente un atome d'hydrogène, un groupe alcoyle en $C_1$ à $C_5$, un groupe aryle ou aralcoyle, un groupe acyle en $C_1$ à $C_3$ ou un groupe benzoyle,

avec cette précision que,

lorsque

$R_3$ représente un atome d'hydrogène, un groupe hydroxyle, un groupe acyloxy ou benzoyloxy,

$R_4$ est un atome d'hydrogène ou un groupe alcoyle et

lorsque

$R_4$ représente un atome d'hydrogène, un atome d'halogène, un groupe hydroxyle, un groupe acyloxy, benzoyloxy ou azido,

$R_3$ est un atome d'hydrogène,

par réaction d'alcaloïdes de type morphinane de formule générale (I)

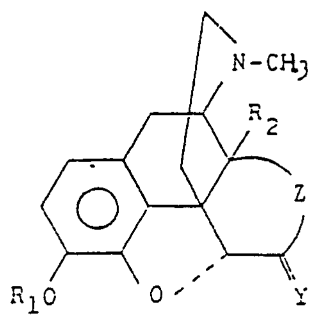

(I)

où Z, Y, $R_1$ et $R_2$ sont tels que définis ci-dessus — avec du phosgène ou diphosgène à une température de 0-120 °C dans un solvant puis par hydrolyse du dérivé N-chlorocarbonyl-N-desméthyle apparu de formule générale (II)

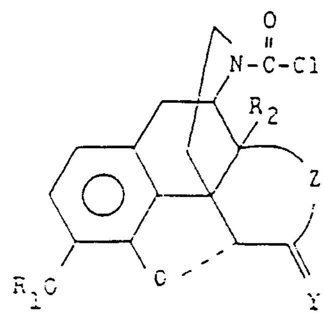

(II)

où Z, Y, $R_1$ et $R_2$ ont la signification donnée ci-dessus avec un acide minéral, caractérisé en ce qu'on conduit la réaction avec du phosgène ou du diphosgène dans le 1,2-dichloroéthane en présence de carbonates alcalins ou de carbonates acides alcalins et que, sans l'isoler, on chauffe le dérivé N-chlorocarbonyl-N-desméthyl apparu de formule générale (II) avec de l'acide chlorhydrique à 5 %.

2. Procédé selon la revendication 1, caractérisé en ce que le rapport molaire des alcaloïdes de type morphinane de formule générale (I) et du phosgène/diphosgène s'élève à 1 : 3-1 : 20, de préférence 1 : 10 et en ce que celui des alcaloïdes de type morphinane de formule générale (I) et des carbonates alcalins/carbonates acides alcalins s'élève à 1 : 0,5-1 : 2 de préférence à 1 : 2.

3. Procédé selon la revendication 1, caractérisé en ce qu'on conduit la réaction avec du diphosgène à une température de 20-120 °C, de préférence 80 °C.

4. Procédé selon la revendication 1, caractérisé en ce qu'on utilise comme carbonate alcalin le carbonate de sodium ou le carbonate de potassium et comme carbonate acide alcalin, le carbonate acide de sodium ou le carbonate acide de potassium.